# EUROPEAN PATENT SPECIFICATION

(11) **EP 0 788 487 B1**
(45) Date of publication and mention of the grant of the patent: **25.07.2001**
(21) Application number: 95937820.9
(22) Date of filing: 23.10.1995
(51) Int. Cl.: C07D 257/04, A61K 31/41

(54) **PROCESS FOR THE PREPARATION OF TETRAZOLES**
VERFAHREN ZUR HERSTELLUNG TETRAZOLE
PROCEDE DE PREPARATION DE TETRAZOLES

(30) Priority: 27.10.1994 CH 322194
(43) Date of publication of application: 13.08.1997
(73) Proprietor: Novartis AG, 4058 Basel (CH); Novartis-Erfindungen Verwaltungsgesellschaft m.b.H., 1235 Wien (AT)
(72) Inventor: ZERGENYI, Janos, CH-4411 Seltisberg (CH)
(86) International application number: EP9504143
(87) International publication number: WO9613489

(56) References cited:
- EP-A- 0 540 356
- EP-A- 0 550 313
- DE-A- 4 313 747

## Description

The invention relates to a process for the preparation of N-protected 4-methyl-2'-(5H-tetrazolyl)biphenyl, to novel target compounds, and to novel starting compounds.

The novel substance class of angiotensin II antagonists opens up novel treatment methods, for example in the treatment of high blood pressure etc. Many of these compounds contain as structural element the 2'-(5H-tetrazolyl)-4-biphenylylmethylene group of the formula 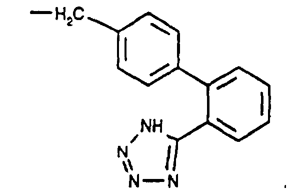 5-(4'-Methylbiphenyl-4-yl)tetrazole and N-protected derivatives thereof are important building blocks in the synthesis of corresponding angiotensin II antagonists. Various efforts have been made to synthesize this structural element.

According to the EP Patent Application having the publication No. 253310 A2 or 324377 A2, the preparation proceeds, for example, from 4'-methyl-2-cyanobiphenyl only via a complicated multi-stage process.

U.S. Patent No. 5,039,814 discloses a route for the preparation, inter alia, of free or 2-tritylated 5-[(4'-methyl)biphenyl-4-yl]tetrazole. To this end, for example, 2-trityl-5-phenyltetrazole is first ortho-lithiated and then reacted with a 4-halotoluene by means of a transmetallation, if desired, the reaction advantageously being carried out in the presence of a catalyst. A disadvantage of this reaction is regarded to be the use of organolithium compounds, which can only be handled on a large scale using complicated safety precautions. Moreover, the use of these organolithium compounds clearly makes the production process more expensive, in particular when transmetallation takes place and the reaction is carried out in the presence of a phosphinated nickel or palladium catalyst. Furthermore, a protective group must not be used which has a hydrogen atom in the alpha-position to the tetrazole nitrogen atom, since this is preferably lithiated and thus the yield of this synthesis variant is clearly reduced.

The EP Patent Application having the publication No. 540356 A2 describes a Grignard reaction in which 5-(2-fluorophenyl)-1 H-tetrazole is reacted with a phenylmagnesium halide, for example p-methylphenylmagnesium bromide. In this context, in many places the use and also the production and disposal of fluorine compounds is no longer appropriate for ecological reasons. Moreover, this synthesis requires approximately 3 equivalents of Grignard reagent; it must furthermore be carried out under reflux temperatures.

The European Patent Application having the publication No. 550313 A1 discloses, for example, a route for the preparation of N-protected 5-(4'-methylbiphenyl-2-yl)tetrazoles. In this process N-protected 2-iodophenyltetrazoles and 2-bromophenyltetrazoles are reacted with toluene derivatives metallated in position 4 by magnesium or zinc, or corresponding tolylboric acid compounds. The use of the bromine and iodine compounds concerned is not very suitable for large-scale use for reasons of cost. Moreover, the regeneration of the resulting bromides or iodides from waste waters is very complicated and cannot be justified for economic and ecological reasons. If appropriate zinc compounds or boric acid derivatives are used as organometallic compounds, provision likewise has to be made for a complicated separation of the metals from the waste waters. When using corresponding magnesium compounds, the yield obtained is unsatisfactory.

A further route for the preparation of 2-cyano-4'-methylbiphenyl via a Grignard reaction is revealed in the EP Patent Application having the publication *No. 566468 A2.* In this process, a 2-halobenzonitrile is treated with a 4-phenylmagnesium halide in the presence of a manganese salt. The formation of corresponding tetrazole derivatives starting from appropriate cyano compounds turns out to be complicated, since tetrazole formation often only takes place with tin azides (e.g. tributyltin azide) in relatively high yields for reasons of steric hindrance. Because of their high ecotoxicity, these azides can be handled on a large scale only with complicated safety precautions. In addition, the removal of tin compounds obtained as impurities from the final product proves very complicated.

The aim of the present invention consists in providing a novel synthesis for the preparation of N-protected 4-methyl-2'-(5H-tetrazolyl)biphenyl, which, independently of the nature of the protective group, besides high yields does not have the disadvantages described above and moreover guarantees a minimization of the ecological pollution of the environment, is economically justifiable, and leads to largely isomerically pure target products and to products of high crystallizability.

It was possible to achieve this object by means of the present invention. The present invention relates to a process for the preparation of N-protected 4-methyl-2'-(5H-tetrazolyl)-biphenyl of the formula (I) 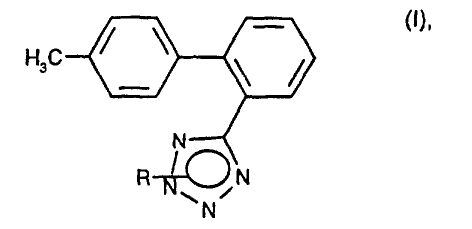 in which R is a tetrazole protective group,
wherein a compound of the formula (II) 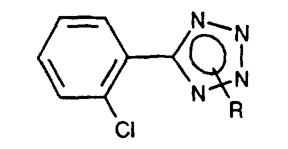 in which R is a tetrazole protective group, is reacted with a compound of the formula (III) 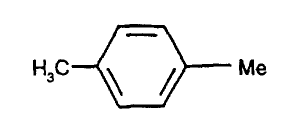 in which Me is a metallic radical, in the presence of a transition metal catalyst.

The tetrazole protective group R should be stable to the organometal reagents used, adequately resistant to brominating agents (as in the next step or in one of the following steps in the reaction sequence for the preparation of angiotensin II antagonists a bromination is carried out) and also easy to introduce and easily removable. Suitable protective groups are, for example, lower alkyl, lower alkyl which (i) is monosubstituted or polysubstited by aryl, or (ii) is substituted by aryl-lower alkoxy, lower alkoxy or lower alkylthio, 2-tetrahydropyranyl, allyl and silyl. Aryl is, for example, phenyl or pyridyl, each of which, independently of one another, is unsubstituted or monosubstituted or polysubstituted, e.g. di- or trisubstituted, e.g. by lower alkyl or lower alkoxy. Silyl is, for example, tri-lower alkylsilyl. Examples of appropriate protective groups which may be mentioned are tert-butyl, benzyl, p-methoxybenzyl, 2-phenyl-2-propyl, diphenylmethyl, di(p-methoxyphenyl)methyl, trityl, (p-methoxyphenyl)diphenylmethyl, diphenyl(4-pyridyl)methyl, benzyloxymethyl, methoxymethyl, ethoxymethyl, methylthiomethyl, 2-tetrahydropyranyl, allyl and tri(m)ethylsilyl.

Preferred protective groups are, for example, benzyl, p-methoxybenzyl and diphenylmethyl; 2-phenyl-2-propyl is particularly preferred.

The protective group R is localized in the 1-position or preferably in the 2-position of the tetrazole ring.

A metallic radical Me is, for example, -Mg-Hal, Hal being halogen, such as chlorine, bromine or iodine. Hal is preferably bromine.

Suitable transition metal catalysts are, for example, corresponding manganese, cobalt, palladium and, primarily, nickel catalysts, such as nickel(ll) halide, e.g. chloride, or nickel acetylacetonate, preferably in the presence of a complexing agent, such as a phosphine, e.g. triphenylphosphine. The catalyst (in particular an Ni salt) is advantageously pretreated with a reducing agent, such as a hydride which, if desired, is complex, e.g. dibutylaluminium hydride or diisobutylaluminium hydride, or a methylmagnesium halide, e.g. methylmagnesium chloride, corresponding Ni(0) catalysts, such as Ni[P(C₆H₅)₃]₄, resulting. Preferably, systems comprising nickel acetylacetonate, triphenylphosphine and diisobutylaluminium hydride are used. Advantageously, 1 to 10 mol %, primarily 2 to 7 mol %, of catalyst are employed per mole of compound of the formula (II).

The phenyl-phenyl coupling according to the invention is carried out, for example, in an inert solvent in a temperature range from approximately 0°C up to the boiling point of the solvent or solvent mixture, e.g. approximately between 20°C and approximately 80°C. Suitable inert solvents are, for example, aliphatic or aromatic hydrocarbons, such as a lower alkyl benzene, or ethers, such as cyclic ethers, e.g. hexane, toluene, a xylene, dioxane or tetrahydrofuran, or mixtures thereof. A molar ratio of compound (II) to compound (III) is, for example, approximately 1.5 to approximately 0.25, primarily approximately 1.0 to approximately 0.5.

Advantageously, the reaction is carried out in an aromatic hydrocarbon, such as a lower alkyl benzene, e.g. toluene or a xylene, an ether, such as a cyclic ether, e.g. tetrahydrofuran, or a mixture thereof. Particularly advantageously, the reaction according to the invention proceeds in a mixture of lower alkyl benzene, in particular toluene or a xylene, and a cyclic ether, in particular tetrahydrofuran. Advantageously, 0.5 to 3 litres, primarily 1 to 2 litres, of solvent are employed per mole of compound of the formula (II).

It is known to prepare biaryls in the presence of transition metal catalysts starting from aryl halides and metallated aryls. It is furthermore known that appropriate bromides or iodides are employed as aryl halides, since halides of this type have a better reactivity compared with corresponding chlorides. If, for example, the reaction conditions as are described in EP 550313 A1 are applied to starting substances of the formulae (II) and (III), significantly lower yields are obtained than when using the inventive conditions of the present process. All the more surprising is the finding that the inventive use of compounds of the formula (II) leads to higher yields of products of the formula (I), compared with the methodology according to EP 550313, in which corresponding iodine derivatives were employed.

The compounds of the formula (II) can be prepared by introduction of the protective group R into the compound of the formula (IV) 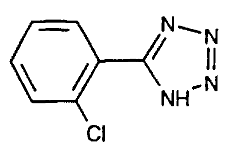 or a proton tautomer thereof.

The compound of the formula (IV) can exist as proton tautomers. The hydrogen atom of the tetrazole ring can be bonded to different N atoms of this ring. Corresponding tautomers are in equilibrium with one another.

If activated alcohols of the type R-OH (V), e.g. compounds of the formula R-X (Va) in which X is halogen or sulfonyloxy, e.g. methanesulfonyioxy, are employed as reagents for the introduction of the protective group, the reaction can be carried out in the presence of a base. Tetrazoles of the formula (IV) are particularly preferably reacted with alcohols of the formula (V) in the presence of an acid.

Possible bases are, for example, alkali metal hydroxides, hydrides, amides, alkoxides, carbonates, triphenylmethylides, di-lower alkylamides, aminoalkylamides or lower alkyl-silylamides, naphthalenamines, lower alkylamines, basic heterocycles, ammonium hydroxides, and also carbocyclic amines. Examples which may be mentioned are sodium hydroxide, hydride or amide, sodium (m)ethoxide, potassium tert-butoxide or carbonate, lithium triphenylmethylide or diisopropylamide, potassium 3-aminopropylamide or bis(trimethylsilyl)amide, dimethylaminonaphthalene, di- or triethylamine, or ethyldiisopropylamine, N-methylpiperidine, pyridine, benzyltrimethylammonium hydroxide, 1,5-diazabicyclo[4.3.0]non-5-ene (DBN) and 1,8-diazabicyclo[5.4.0]undec-7-ene (DBU).

Suitable acids are, for example, inorganic acids, such as mineral acids, e.g. sulfuric acid, a phosphorus or halohydric acid, or organic acids. Organic sulfonic acids are preferred, such as unsubstituted or substituted, e.g. by halogen, [(C₁-C₄)-]alkane or arylsulfonic acids e.g. methane- or toluenesulfonic acid.

If acids are employed in the reaction of the compound of the formula (IV) with compounds of the formula (V), the protective group R is surprisingly preferably bonded in position 2 of the tetrazole ring, while, in contrast, when using bases, the protective group is bonded both in position 1 and in position 2. The use of acids unexpectedly causes the formation of an essentially isomerically pure form. The choice of the condensing agent thus plays a significant part for the synthesis of an essentially isomerically pure form. For example, products having an isomeric purity of more than 75%, advantageously of more than 90%, are thus obtained.

The protective group can also be introduced by addition of a corresponding olefinic derivative derived from the alcohol of the formula (V). For example, the 2-pyranyl protective group is thus introduced by acid-catalysed reaction with dihydropyran, and the 2-phenyl-2-propyl group by reaction with 2-phenylpropene. Accordingly, the present invention also relates to the preparation of compounds of the formula (IV) shown above.

The invention furthermore relates to the following process sequence for the preparation of compounds of the formula (I), wherein
(a) a protective group R is introduced into the compound of the formula (IV) or a proton tautomer thereof and then
(b) a resultant compound of the formula (II) is reacted with a compound of the formula (III) in which Me is a metallic radical, in the presence of a transition metal catalyst.

The present invention further relates to new compounds of the formula (I) and new starting materials which can be used for the preparation of compounds of the formula (I), i.e. in particular new compounds of the formulae (II) and (IV), and the use of compounds of the formulae (II) and (IV) for the preparation of compounds of the formulae (I) and also for the preparation of angiotensin (II) antagonists which contain the 2'-(5H-tetrazolyl)-4-biphenylylmethylene group. The methyl group in compounds of the formula (I) can thus be halogenated by treatment with a halogenating reagent. For example, the methyl group can be brominated, a customary brominating agent being employed, e.g. N-bromosuccinimide, advantageously in the presence of an azo compound, e.g. azobisisobutyronitrile. The preparation of angiotensin (II) antagonists of this type is described, for example, in the European Patent Application having the publication No. 253310.

The following novel compounds are prepared by the process of claim 1:
5-[2-(4'-methylbiphenylyl)]-2-[(2-phenyl)-2-propyl]tetrazole;
5-[2-(4'-methylbiphenylyl)]-2-(diphenylmethyl)tetrazole;
5-[2-(4'-methylbiphenylyl)]-2-(4-methoxybenzyl)tetrazole;
5-(2-chlorophenyl)-2-[(2-phenyl)-2-propyl]tetrazole;
5-(2-chlorophenyl)-2-(diphenylmethyl)tetrazole;
5-(2-chlorophenyl)-2-(4-methoxybenzyl)tetrazole.

The following examples serve to illustrate the present invention.

### Example 1: 5(-2-Chlorophenyl)-2-(4-methoxybenzyl)tetrazole

2 ml of methanesulfonic acid are added to a suspension of 36 g of 5-(2-chlorophenyl)-tetrazole in 150 ml of toluene and a solution of 28 g of 4-methoxybenzyl alcohol in 60 ml of toluene is added dropwise at a temperature of approximately 110°C with azeotropic separation of water. The solution is additionally stirred for about 10 minutes, then cooled to room temperature, diluted with 100 ml of toluene, washed, first with water then with a solution of sodium hydrogen carbonate, and concentrated to dryness. According to the NMR spectrum, the crude product (yield 61 g; 100% of theory) contains about 90% of 5-(2-chlorophenyl)-2-(4-methoxybenzyl)tetrazole and about 10% of the 1-(4-methoxybenzyl) isomer. After recrystallization from isopropanol, 51.2 g (85 % of theory) of the isomer-free 5-(2-chlorophenyl)-2-(4-methoxybenzyl)tetrazole are obtained, m.p.. 77-79°C (uncorrected).

The following can be prepared, for example, in an analogous manner:
(a) 5-(2-Chlorophenyl)-2-benzyltetrazole (crude yield about 100% of theory)
   According to the NMR spectrum, the crude product contains about 75% of the 2-benzyl isomer and about 25% of the 1-benzyl isomer. Oil, crystallizes on allowing to stand. [thin-layer chromatography (TLC); silica gel plate; mobile phase: hexane/ethyl acetate 2:1; R_{f} = 0.28 (1-benzyl isomer); R_{f} = 0.5 (2-benzyl isomer)]
(b) 5-(2-Chlorophenyl)-2-[(2-phenyl)-2-propyl]tetrazole
   Yield about 100%, oily, isomerically pure according to TLC [silica gel plate, mobile phase: hexanelethyl acetate 2:1; R_{f}: about 0.66]
(c) 5-(2-Chlorophenyl)-2-(diphenylmethyl)tetrazole
   Crude yield about 100%, oily, isomerically pure according to TLC [mobile phase: hexane/ethyl acetate 2:1; R_{f}: about 0.59].

### Example 2: 5-(2-Chlorophenyl)-1-benzyltetrazole and 5-(2-chlorophenyl)-2-benzyltetrazole

10.6 g of benzyl bromide is added dropwise at a temperature of about 60°C to a well-stirred mixture of 9 g of 5-(2-chlorophenyl)tetrazole, 30 ml of toluene, 0.4 g of tetrabutylammonium hydrogen sulfate and 30 g of 2N aqueous sodium hydroxide solution. The solution is additionally stirred for about 1 hour, then cooled to room temperature, and the toluene phase is separated off, washed with dilute sodium hydroxide solution in water and evaporated to dryness. According to the NMR spectrum, the crude product (11.1 g; 82% of theory) contains about 40% of the 2-benzyl isomer and about 60% of the 1-benzyl isomer. Oil, crystallizes on allowing to stand. Thin-layer chromatography [silica gel plate; mobile phase: hexane/ethyl acetate 2:1; R_{f} = 0.28 (1-benzyl isomer); R_{f} = 0.5 (2-benzyl isomer)]

### Example 3: 5-(2-Chlorophenyl)-2-[(2-phenyl)-2-propyl]tetrazole

1 ml of methanesulfonic acid is added to a suspension of 18 g of 5-(2-chlorophenyl)tetrazole in 150 ml of toluene and a solution of 12.2 g of 2-phenylpropene in 30 ml of toluene is added dropwise at a temperature of approximately 110°C. The solution is additionally stirred for about 10 minutes, then cooled to room temperature, diluted with 100 ml of toluene, washed, first with water then with a solution of sodium hydrogen carbonate, and concentrated to dryness. The crude product (yield 27.5 g; 92% of theory) is oily and isomerically pure according to thin-layer chromatography (cf. Example 1b).

### Example 4: 5-[2-(4'-Methylbiphenylyl)]-2-(4-methoxybenzyl)tetrazole

A solution of 4-tolylmagnesium bromide is prepared at 60°C from 7 g of magnesium in 50 ml of tetrahydrofuran and 44.6 g of 4-bromotoluene in 90 ml of toluene. 49 g of 5-(2-chlorophenyl)-2-(4-methoxybenzyl)tetrazole at 20-30°C are added to a mixture of 50 ml of toluene, 2.3 g of nickel acetylacetonate, 8.8 g of triphenylphosphine and 17.5 ml of an about 20 % solution of diisobutylaluminium hydride in toluene and subsequently the 4-tolylmagnesium bromide solution prepared above is added dropwise, then the mixture is additionally stirred for about 20 hours. 175 ml of toluene and 175 ml of 2N hydrochloric acid are then added, and the toluene phase is separated off and concentrated. The evaporation residue is crystallized from isopropanol. Yield: 45 g, 78% of theory; m.p. 75-77°C. A further 3.9 g (7% of theory) of the product can be obtained from the mother liquor by chromatography on silica gel. Total yield: 85% of theory.

The following can be prepared, for example, in an analogous manner:
(a) 5-[2-(4'-Methylbiphenylyl)]-2-benzyltetrazole
   Starting from crude 5-(2-chlorophenyl)-2-benzyltetrazole. Crude yield about 100%. [TLC; silica gel plate; mobile phase: hexane/ethyl acetate 2:1; R_{f} = 0.2 (1-benzyl isomer); R_{f} = 0.38 (2-benzyl isomer)].
   According to the NMR spectrum, the crude product contains about 75% of the 2-benzyl isomer and about 25% of the 1-benzyl isomer.
(b) 5-[2-(4'-Methylbiphenylyl)]-2-[(2-phenyl)-2-propyl]tetrazole starting from 5-(2-chlorophenyl)-2-[(2-phenyl)-2-propyl]tetrazole.
   Yield: 79% of theory; m.p. 102-103 (uncorr.)
(c) 5-[2-(4'-Methylbiphenylyl)]-2-(diphenylmethyl)tetrazole starting from 5-(2-chlorophenyl)-2-diphenylmethyltetrazole.
   Crude yield about 100%; TLC; silica gel plate; mobile phase: toluene; R_{f} = 0.3.

### Example 5: 5-[2-(4'-Methylbiphenylyl)]-2-(2-phenyl-2-propyl)tetrazole

A solution of 4-tolylmagnesium bromide is prepared at 60° from 21.6 g of magnesium in 170 ml of tetrahydrofuran and 135.5 g of 4-bromotoluene in 300 ml of toluene.

164.3 g of 5-(2-chlorophenyl)-2-(2-phenyl-2-propyl)tetrazole at 20-30° are added to a mixture of 180 ml of toluene, 6.8 g of nickel acetylacetonate, 26.4 g of triphenylphosphine and 54 ml of a solution of diisobutylaluminium hydride in toluene and subsequently the 4-tolyl-magnesium bromide solution prepared above is added dropwise. The mixture is then additionally stirred for about 20 hours. 480 ml of toluene and 480 ml of 2N hydrochloric acid are added and the toluene phase is separated off and concentrated in vacuo. The evaporation residue is crystallized from isopropanol. 5-[2-(4'-Methylbiphenylyl)]-2-(2-phenyl-2-propyl)tetrazole is thus obtained.
Yield (without working up the mother liquor): 162.8 g (83.5% of theory); m.p. 102-103°.

## Claims

1. A process for the preparation of N-protected 4-methyl-2'-(5H-tetrazolyl)biphenyl of the formula (I) 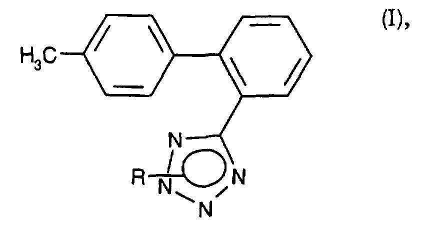 in which R is a tetrazole protective group,
wherein
a compound of the formula (II) 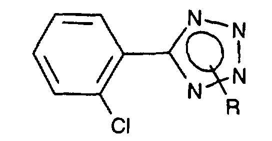 in which R is a tetrazole protective group, is reacted with a compound of the formula (III') 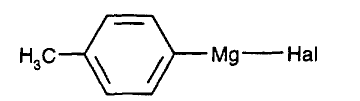 in which Hal is halogen, in the presence of a transition metal catalyst.

2. A process according to claim 1, wherein the transition metal catalysts employed are manganese, cobalt, palladium or nickel catalysts.

3. A process according to claim 2, wherein the transition metal catalyst employed is a nickel catalyst.

4. A process according to claim 3, wherein an Ni salt is pretreated with a reducing agent, such as a hydride which, if desired, is complex, e.g. dibutylaluminium hydride, or a methylmagnesium halide, e.g. methylmagnesium chloride, and employed in the presence of a complexing agent, such as a phosphine, e.g. triphenylphosphine.

5. A process according to any one of claims 1-4, wherein Ni[P(C₆H₅)₃]₄ is use as catalyst.

6. A process according to any one of claims 1-5, wherein the reaction is carried out in aliphatic or aromatic hydrocarbon, such as hexane, toluene or a xylene, or an ether, such as tetrahydrofuran, as a solvent or a mixture thereof.

7. A process according to claim 6, wherein the reaction is carried out in a mixture of toluene and tetrahydrofuran.

8. A process according to claim 7, wherein 0.5 to 3 litres, in particular 1 to 2 litres, of solvent are employed per mole of compound of the formula (II).

9. A process according to any one of claims 1-8, wherein a possible protective group R is lower alkyl, lower alkyl which (i) is monosubstituted or polysubstituted by aryl, or (ii) is substituted by aryl-lower alkoxy, lower alkoxy or lower alkylthio, 2-tetrahydropyranyl, allyl or silyl.

10. A process according to claim 9, wherein a possible protective group R is tert-butyl, benzyl, p-methoxybenzyl, 2-phenyl-2-propyl, diphenylmethyl, di(p-methoxyphenyl)methyl, trityl, (p-methoxyphenyl)diphenylmethyl, diphenyl(4-pyridyl)methyl, benzyloxymethyl, methoxymethyl, ethoxymethyl, methylthiomethyl, 2-tetrahydropyranyl, allyl or tri(m)ethylsilyl.

11. A process according to claim 10, wherein a possible protective group R is benzyl, p-methoxybenzyl, diphenylmethyl or 2-phenyl-2-propyl.

12. A process according to any one of claims 1-11, wherein the protective group R is localized in the 1-position or preferably in the 2-position of the tetrazole ring.

13. A process according to any one of claims 1-12, wherein the starting material used is a compound of the formula (III) in which Hal is bromine.

14. A process according to any one of claims 1 to 13 for the preparation of N-protected 4-methyl-2'-(5H-tetrazolyl)biphenyl of the formula (I) 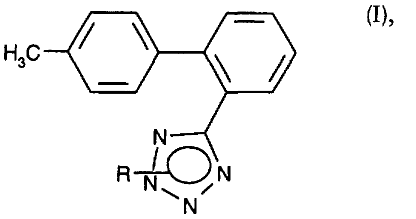 in which R is a tetrazole protective group,
wherein
(a) the protective group R is introduced into the compound of the formula (IV) 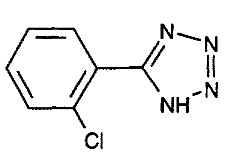 or a proton tautomer thereof and
(b) a resultant compound of the formula (II) 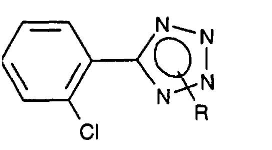 in which R is a tetrazole protective group, is reacted with a compound of the formula (III') 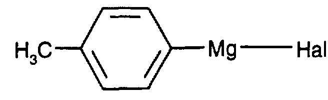 in which Hal is halogen, in the presence of a transition metal catalyst.

15. A process according to claim 14, wherein the starting material is a compound of the formula (IV) and this is reacted with 2-phenylpropene in the presence of an acid to give a compound of the formula (II) in which R is 2-phenyl-2-propyl.

## Patentansprüche

1. Verfahren zur Herstellung von N-geschütztem 4-Methyl-2'-(5H-tetrazolyl)biphenyl der Formel (I) 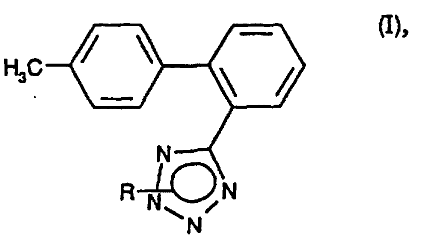 worin R für eine Tetrazolschutzgruppe steht,
worin eine Verbindung der Formel (II) 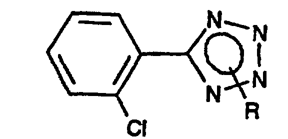 worin R für eine Tetrazolschutzgruppe steht, mit einer Verbindung der Formel (III') 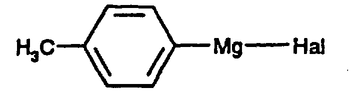 worin Hal für Halogen steht, in Gegenwart eines Übergangsmetallkatalysators umgesetzt wird.

2. Verfahren nach Anspruch 1, worin die verwendeten Übergangsmetallkatalysatoren Mangan, Kobalt, Palladium oder Nickelkatalysatoren sind.

3. Verfahren nach Anspruch 2, worin der verwendete Übergangsmetallkatalysator ein Nickelkatalysator ist.

4. Verfahren nach Anspruch 3, worin ein Ni Salz mit einem Reduktionsmittel vorbehandelt wird, wie einem Hydrid, das erforderlichenfalls komplexiert ist, beispielsweise Dibutylaluminiumhydrid, oder ein Methylmagnesiumhalogenid, beispielsweise Methylmagnesiumchlorid, und in Gegenwart eines Komplexicrungsmittels. wie einem Phosphin, beispielsweise Triphenylphosphin.

5. Verfahren nach einem der Ansprüche 1 bis 4, worin Ni[P(C₆H₅)₃]₄ als Katalysator verwendet wird.

6. Verfahren nach einem der Ansprüche 1 bis 5, worin die Umsetzung in einem aliphatischen oder aromatischen Kohlenwasserstoff, wie Hexan, Toluol oder einem Xylol oder einem Ether, wie Tetrahydrofuran als Lösemittel oder einem Gemisch hiervon ausgeführt wird.

7. Verfahren nach Anspruch 6, worin die Umsetzung in einem Gemisch aus Toluol und Tetrahydrofuran ausgeführt wird.

8. Verfahren nach Anspruch 7, worin 0,5 bis 3 Liter, insbesondere 1 bis 2 Liter Lösemittel pro Mol der Verbindung der Formel (II) verwendet werden.

9. Verfahren nach einem der Ansprüche 1 bis 8, worin eine mögliche Schutzgruppe R für Niederalkyl, Niederalkyl, das (i) monosubstituiert oder polysubstituiert ist durch Aryl oder (ii) durch Arylniederalkoxy Niederalkoxy oder Niederalkylthio, 2-Tetrahydropyranyl, Allyl oder Silyl substituiert ist.

10. Verfahren nach Anspruch 9, worin eine mögliche Schutzgruppe steht für tert-Butyl, Benzyl, p-Methoxybenzyl, 2-Phenyl-2-propyl, Diphenylmethyl, Di(p-methoxyphenyl)methyl, Trityl, (p-Methoxyphenyl)diphenylmethyl, Diphenyl(4-pyridyl)methyl, Benzyloxymethyl, Methoxymethyl, Ethoxymethyl, Methylthiomethyl, 2-Tetrahydropyranyl, Allyl, Trimethylsilyl oder Triethylsilyl.

11. Verfahren nach Anspruch 10, worin eine mögliche Schutzgruppe R für Benzyl, p-Methoxybenzyl, Diphenylmethyl oder 2-Phenyl-2-propyl steht.

12. Verfahren nach einem der Ansprüche 1 bis 11, worin die Schutzgruppe R an der Position 1 oder vorzugsweise an der Position 2 des Tetrazolrings liegt.

13. Verfahren nach einem der Ansprüche 1 bis 12, worin das verwendete Ausgangsmaterial eine Verbindung der Formel III ist, worin Hal für Brom steht

14. Verfahren nach einem der Ansprüche 1 bis 13 zur Herstellung von N-geschütztem 4-Methyl-2'-(5H-tetrazolyl)biphenyl der Formel (I) 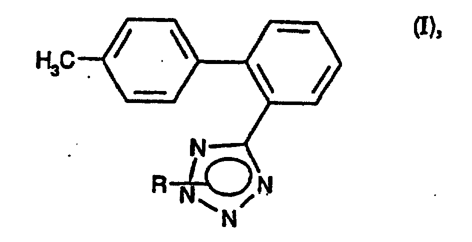 worin R für eine Tetrazolschutzgruppe steht, worin
(a) die Schutzgruppe R in die Verbindung der Formel (IV) 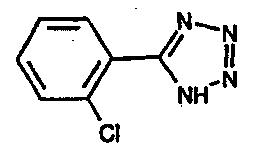 oder ein Protonentautomer hiervon eingeführt wird, und
(b) eine entstehende Verbindung der Formel (II) 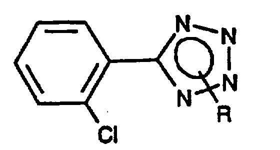 worin R für eine Tetrazolschutzgruppe steht, mit einer Verbindung der Formel (III') 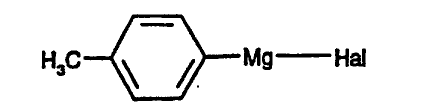 worin Hal für Halogen steht, in Gegenwart eines Übergangsmetallkatalysators umgesetzt wird.

15. Verfahren nach Anspruch 14, worin das Ausgangsmaterial eine Verbindung der Formel (IV) ist und mit 2-Phenylpropen in Gegenwart einer Säure unter Bildung einer Verbindung der Formel (II) umgesetzt wird, worin R für 2-Phenyl-2-propyl steht.

## Revendications

1. Un procédé de préparation de 4-méthyl-2'-(5H-tétrazolyl)biphényle N-protégé de formule (I) 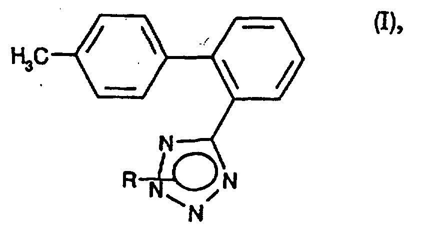 où R signifie un groupe protecteur du groupe tétrazole,
où
un composé de formule (II) 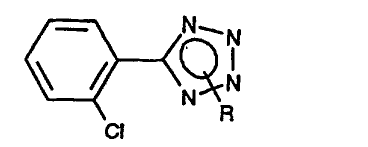 où R signifie un groupe protecteur du groupe tétrazole, est mis à réagir avec un composé de formule (III') 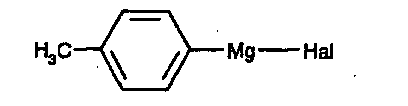 où Hal signifie un halogène, en présence d'un catalyseur de métal de transition.

2. Un procédé selon la revendication 1, où les catalyseurs de métaux de transition utilisés sont les catalyseurs de manganèse, de cobalt, de palladium ou de nickel.

3. Un procédé selon la revendication 2, où le catalyseur de métal de transition utilisé est un catalyseur de nickel.

4. Un procédé selon la revendication 3, où un sel de Ni est prétraité avec un agent réducteur tel qu'un hydrure qui, si nécessaire, est complexe, par exemple l'hydrure de dibutylaluminium, ou un halogénure de méthylmagnésium, par exemple le chlorure de méthylmagnésium, et est utilisé en présence d'un agent complexant tel qu'une phosphine, par exemple la triphénylphosphine.

5. Un procédé selon l'une quelconque des revendications 1-4, où Ni[P(C₆H₅)₃]₄ est utilisé comme catalyseur.

6. Un procédé selon l'une quelconque des revendications 1-5, où la réaction est effectuée dans un hydrocarbure aliphatique ou aromatique tel que l'hexane, le toluène ou un xylène, ou un éther, tel que le tétrahydrofuranne, comme solvant ou un de ses mélanges.

7. Un procédé selon la revendication 6, où la réaction est effectuée dans un mélange de toluène et de tétrahydrofuranne.

8. Un procédé selon la revendication 7, où on utilise de 0,5 à 3 litres, en particulier de 1 à 2 litres de solvant par mole de composé de formule (II).

9. Un procédé selon l'une quelconque des revendications 1-8, où un groupe protecteur R possible est un groupe alkyle inférieur, alkyle inférieur qui (i) est monosubstitué ou polysubstitué par un groupe aryle, ou bien (ii) est substitué par un groupe aryl-alcoxy inférieur, alcoxy inférieur ou alkyl inférieur-thio, 2-tétrahydropyranyle, allyle ou silyle.

10. Un procédé selon la revendication 9, où un groupe protecteur R possible est le groupe tert.-butyle, benzyle, p-méthoxybenzyle, 2-phényl-2-propyle, diphénylméthyle, di(p-méthoxyphényl)méthyle, trityle, (p-méthoxyphényl)diphénylméthyle, diphényl(4-pyridyl)méthyle, benzyloxyméthyle, méthoxyméthyle, éthoxyméthyle, méthylthiométhyle, 2-tétrahydropyranyle, allyle, ou tri(m)éthylsilyle.

11. Un procédé selon la revendication 10, où un groupe protecteur R possible est le groupe benzyle, p-méthoxybenzyle, diphénylméthyle ou 2-phényl-2-propyle.

12. Un procédé selon l'une quelconque des revendications 1-11, où le groupe protecteur R est localisé en position 1 ou de préférence en position 2 du cycle tétrazole.

13. Un procédé selon l'une quelconque des revendications 1-12, où le produit de départ utilisé est un composé de formule (III) où Hal signifie le brome.

14. Un procédé selon l'une quelconque des revendications 1 à 13, pour la préparation de 4-méthyl-2'-(5H-tétrazolyl)biphényle N-protégé de formule (I) 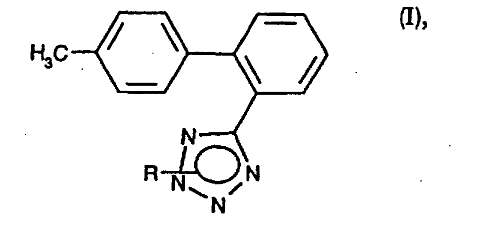 où R signifie un groupe protecteur du groupe tétrazole,
où
(a) le groupe protecteur R est introduit dans le composé de formule (IV) 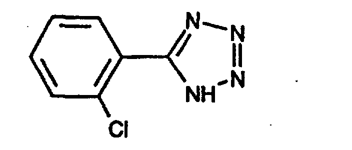 ou un de ses tautomères de proton et
(b) un composé résultant de formule (II) 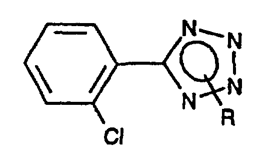 où R signifie un groupe protecteur du groupe tétrazole, est mis à réagir avec un composé de formule (III') 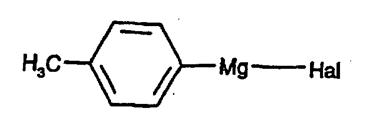 où Hal signifie un halogène, en présence d'un catalyseur de métal de transition.

15. Un procédé selon la revendication 14, où le produit de départ est un composé de formule (IV) et qui est mis à réagir avec le 2-phénylpropène en présence d'un acide, pour donner un composé de formule (II) dans lequel R signifie un groupe 2-phényl-2-propyle.
